# FASCICULE DE BREVET EUROPEEN

(11) **EP 0 545 786 B1**
(45) Date de publication et mention de la délivrance du brevet: **16.10.1996**
(21) Numéro de dépôt: 92403192.5
(22) Date de dépôt: 26.11.1992
(51) Int. Cl.: A61K 7/021, A61K 7/48, A61K 7/035

(54) **Composition cosmétique pour le maquillage et les soins de la peau contenant un composé perfluoroalkyle**
Perfluoralkylverbindungen enthaltende Make-up- und Hautpflegemittel
Skin treatment and make-up composition containing a perfluoroalkyl compound

(30) Priorité: 26.11.1991 FR 9114572
(43) Date de publication de la demande: 09.06.1993
(73) Titulaire: L'OREAL, F-75008 Paris (FR)
(72) Inventeur: Candau, Didier, F-91570 BIEVRES (FR); Mellul, Myriam, F-94240 l'Hay Les Roses (FR)
(74) Mandataire: Stalla-Bourdillon, Bernard

(56) Documents cités:
- US-A- 3 632 744
- DATABASE WPIL Week 8934, Derwent Publications Ltd., London, GB; AN 89-246393
- FEY H. & OTTE I. 'WÖRTEBUCH DER KOSMETIK' 1985 , WISSENSCHAFTLICHE VERLAGSGESELLSCHAFT MBH , STUTTGART

## Description

La présente invention a pour objet une composition cosmétique pour le maquillage ou les soins de la peau contenant une poudre, sous forme compactée ou libre ou bien sous forme d'une dispersion dans un liant approprié, en présence d'un composé perfluoroalkyle.

Les compositions cosmétiques à base de poudre sont essentiellement les fards à paupières, les fards anhydres, les fonds de teint, les sticks, les mascaras, les poudres de nettoyage ou parfumées pour les soins du corps et de la toilette, les crèmes sous forme d'émulsion huile-dans-eau (H/E) ou eau-dans-huile (E/H).

Par l'expression "poudre" telle qu'utilisée selon l'invention, on doit entendre non seulement les charges mais également les pigments.

Parmi les propriétés que doivent présenter les compositions contenant des poudres, les plus importantes sont une bonne adhésion à la surface de la peau, un bon pouvoir couvrant de façon à dissimuler certains défauts ou imperfections de la peau, et un bon pouvoir d'étalement sur la peau.

Il convient par ailleurs qu'au moment de leur préparation, les poudres puissent se disperser aisément dans le véhicule, qu'il soit du type lipophile ou hydrophile.

Il est particulièrement difficile de trouver des poudres susceptibles de remplir toutes ces différentes exigences qui sont souvent contradictoires. Elles sont parfois résolues par l'emploi de mélange de poudres, ce qui permet de trouver un équilibre entre ces différentes propriétés.

Toutefois, cette solution présente l'inconvénient de faire régresser les propriétés intrinsèques des compositions. Si l'on peut améliorer l'étalement d'une composition à base de poudre transparente par l'addition de charge ou de poudre lamellaire, on note cependant une augmentation de la couvrance et par conséquent le risque d'un aspect farineux par application de la composition sur la peau.

Par ailleurs, si l'introduction d'une huile volatile, telle que par exemple une huile de silicone volatile dans un fond de teint très riche en poudre, améliore l'étalement et l'homogénéité de la composition appliquée sur la peau, on observe un toucher gras à l'application.

Il a par ailleurs été décrit dans US-3.632.744, l'amélioration des propriétés cosmétiques d'une composition pulvérulente par enrobage du substrat poudreux à l'aide d'un composé fluoré.

Il a également été décrit dans JP-A-1.180.810 des compositions cosmétiques contenant une poudre enrobée par un composé contenant des groupes polyfluoroalkyles et oxyalkylènes.

A la suite de diverses études, on a constaté de façon surprenante et inattendue qu'il était possible d'améliorer les propriétés des poudres, en particulier d'écoulement et d'étalement dans des compositions cosmétiques de maquillage ou de soin de la peau, et ceci quelque soit le véhicule cosmétique, en utilisant un composé perfluoroalkyle.

On a, en effet, observé que par l'emploi d'une quantité faible de composé perfluoroalkyle à l'état libre, les propriétés intrinsèques des compositions n'étaient pas modifiées mais que du fait des propriétés d'agent de surface du composé perfluoroalkyle, il se produisait une modification importante des caractéristiques d'étalement et d'écoulement des compositions, ce qui améliorait les propriétés cosmétiques.

La présente invention a donc pour objet une composition cosmétique pour le maquillage ou les soins de la peau contenant de 1 à 98 % en poids d'au moins une poudre et au moins 1 % en poids d'un corps gras, ladite composition contenant en outre de 0,05 à 1 % en poids d'un composé perfluoroalkyle dont le radical alkyle à de 4 à 32 atomes de carbone, de préférence de 4 à 22 atomes de carbone, ledit composé perfluoroalkyle étant à l'état libre.

Comme mentionné ci-dessus, les poudres proviennent essentiellement des charges et pigments et se présentent sous forme de particules sphériques, lamellaires, aciculaires, etc... ayant des tailles de particules généralement inférieures à 500 µm et de préférence inférieures à 100 µm.

Parmi les charges des compositions selon l'invention, on peut notamment citer:
- le talc, qui est un silicate de magnésium hydraté utilisé sous forme de particules généralement de dimensions inférieures à 40 µm ;
- les micas, qui sont des aluminosilicates de compositions variées, qui se présentent sous la forme d'écailles ayant des dimensions de 2 à 200 µm, de préférence de 5 à 70 µm et une épaisseur de 0,1 à 5 µm, de préférence de 0,2 à 3 µm. Les micas peuvent être d'origine naturelle (par exemple muscovite, margarite, roscoélithe, lépidolithe, biotite) ou d'origine synthétique ;
- l'amidon, en particulier l'amidon de riz;
- le kaolin qui est un silicate d'aluminium hydraté qui se présente sous la forme de particules de forme isotrope ayant des dimensions généralement inférieures à 30 µm ;
- les oxydes de zinc et de titane, généralement utilisés sous la forme de particules ayant des dimensions ne dépassant pas quelques µm (ou même inférieures à 1 µm dans le cas de l'oxyde de titane) ;
- le nitrure de bore ;
- le carbonate de calcium précipité de dimensions de particules inférieures à 10 µm;
- le carbonate ou le bicarbonate de magnésium ;
- les poudres de polymères de synthèse (éventuellement réticulés) tels que le polyéthylène, les polyesters, les polyamides, par exemple les poudres de nylon ou de poly-p-alanine, de polystyrène, de silicone ou de téflon.

Parmi les pigments, on peut citer :
- le dioxyde de titane (rutile ou anathase) éventuellement traité en surface tel que le produit codifié dans le Color Index sous la référence CI 77891 ;
- les oxydes de fer noir, jaune, rouge et brun, tels que ceux codifiés sous les références CI 77499, 77492, 77491 ; le violet de manganèse ;
- le bleu outremer ;
- l'oxyde de chrome hydraté ;
le bleu ferrique, et tous les pigments minéraux ayant subi un traitement de surface minéral ou organique.

Parmi les pigments organiques, on peut citer les suivants:
- D&C Red n° 19 (CI 45170) ;
- D&C Red n° 9 (CI 15585);
- D&C Red n° 21 (CI 45380);
- D&C Orange n° 4 (CI 15510);
- D&C Orange n° 5 (CI 45370);
- D&C Red n° 27 (CI 45410);
- D&C Red n° 13 (CI 15630);
- D&C Red n° 7 (CI 15850-1);
- D&C Red n° 6 (CI 15850-2);
- D&C Yellow n° 5 (CI 19140);
- D&C Red n° 36 (CI 12085);
- D&C Orange n° 10 (CI 45125);
- D&C Yellow n° 6 (CI 15985);
- D&C Red n° 30 (CI 73360);
- D&C Red n° 3 (CI 45430);
- le noir de carbone (CI 77266); et
- les laques à base de carmin de cochenille (CI 75470).

Les pigments peuvent être également des pigments nacrés choisis parmi :
- les pigments nacrés blancs, tels que le mica recouvert d'oxyde de titane ou l'oxychlorure de bismuth,
les pigments nacrés colorés, tels que le micatitane avec des oxydes de fer, le micatitane avec du bleu ferrique ou de l'oxyde de chrome, le micatitane avec un pigment organique de type précité, ainsi que ceux à base d'oxychlorure de bismuth,
et tous les pigments organiques ayant subi un traitement de surface minéral ou organique.

Le composé perfluoroalkyle est choisi parmi ceux ayant les formules (I) et (II) suivantes :
1) 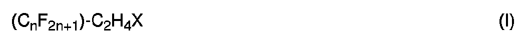 dans laquelle :
   le radical CₙF₂ₙ₊₁ est linéaire ou ramifié,
   n est compris entre 4 et 16,
   X représente un radical choisi parmi :
      (i) -C0₂Y
      (ii) -SO₃Y
         Y représentant un atome d'hydrogène, un métal alcalin ou un groupe aminé tel qu'un groupe ammonium
      (iii) -(OC₂H₄)m-OH
         m étant compris entre 2 et 100, de préférence entre 4 et 40. 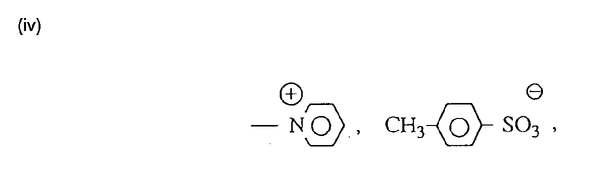 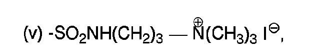 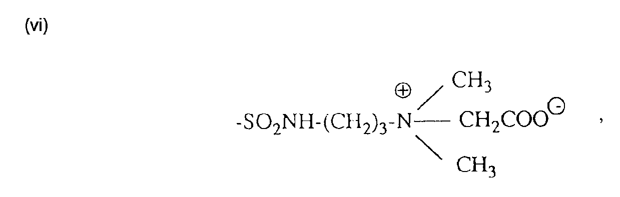 et 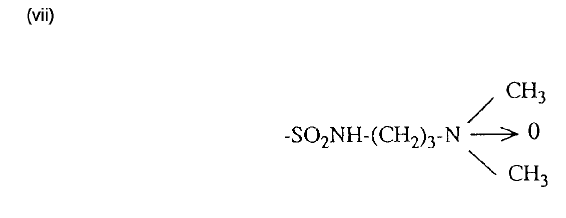
2) 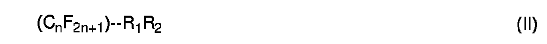 dans laquelle
   le radical CₙF₂ₙ₊₁ est linéaire ou ramifié,
   n étant compris entre 4 et 16,
   R₁ est un reste S0₃, S0₂ ou CO₂
   R₂ est un reste choisi parmi :
      (i) NH₄^{⊕},
      (ii) - N(R₃)CH2-CO₂⊖ X⊕
         R₃ étant un radical alkyle en C₁-C_{4 '} et
         X est un atome d'hydrogène ou un métal alcalin,
      (iii) -NH(CH₂)p 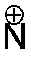 (R₃)₃ l^{⊖}
         p étant 1, 2, 3 ou 4 et
         R₃ étant tel que défini ci-dessus,
      (iv) -N(R₃)(CH₂CH₂0)-Y
         Y étant un atome d'hydrogène ou un radical alkyle en C₁-C₄, et
         R₃ étant tel que défini ci-dessus,
      (v) - 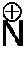 (R₃)₃
         R₃ étant tel que défini ci-dessus.

Parmi les composés perfluoroalkyles de formule (I), on peut notamment citer ceux correspondant aux formules suivantes : 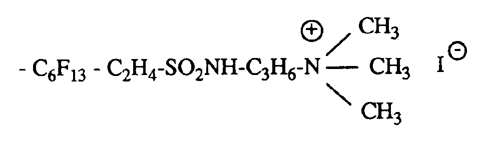 vendu sous la dénomination de "FORAFAC 1179" par la Société ATOCHEM ; 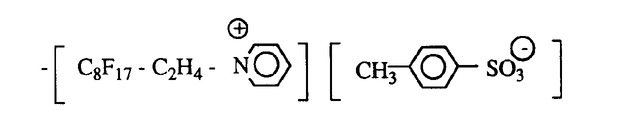 vendu sous la dénomination de "FORAFAC 1098" par la Société ATOCHEM.

Parmi les composés perfluoroalkyles de formule (II), on peut notamment citer : 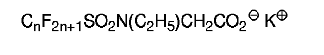
n étant ≈ 8
   vendu sous la dénomination de "FLUORAD FC 129" par la Société 3M ; 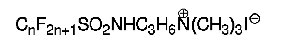
n étant", 8
   vendu sous la dénomination de "FLUORAD FC 135" par la Société 3M ; 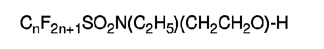
n étant ≈ 8
   vendu sous la dénomination de "FLUORAD FC 170C" par la Société 3M ; 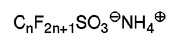
n≈10
   vendu sous la dénomination de "FLUORAD FC 120" par la Société 3M.

Dans les compositions cosmétiques, selon l'invention, le composé perfluoroalkyle est généralement présent en une proportion qui est fonction de la concentration en poudre de la composition.

La proportion de composé perfluoroalkyle est de préférence comprise entre 0,1 et 0,5 % en poids par rapport au poids total de la composition.

Les compositions cosmétiques, selon l'invention, sont notamment des fards à paupières, des fards à joues, des eye-liners, des mascaras, des fonds de teint, des "blush", des crèmes teintées, des rouges à lèvres, des sticks pour les lèvres ou encore des sticks anti-cernes.

De façon préférentielle, les compositions cosmétiques, selon l'invention, sont plus particulièrement destinées au maquillage des yeux, c'est-à-dire sous forme de fards à paupières, de mascaras ou d'eye-liners.

Dans ces compositions, la proportion de corps gras est généralement comprise entre 1 et 90 % et de préférence entre 5 et 80 %.

Ces compositions peuvent se présenter sous forme d'une émulsion huile-dans-eau ou eau-dans-huile, ou sous forme d'une suspension en milieu solvant ou encore sous forme solide ou pâteuse anhydre. Les modes opératoires pour la préparation de ces différents types de compositions sont bien connus de l'homme de l'art.

Lorsqu'elles sont utilisées sous forme d'émulsion, les compositions peuvent contenir des agents tensioactifs conventionnels dans l'état de la technique.

Les compositions conformes à la présente invention peuvent également contenir des additifs conventionnels utilisés dans les compositions de maquillage et de soins pour la peau, à savoir des adoucissants, des conservateurs, des séquestrants, des parfums, des épaississants, des agents de cohésion, des polymères ainsi que des agents alcalini- sants ou acidifiants ainsi que des hydratants.

Les épaississants sont choisis de préférence parmi les gommes arabique, de guar ou de caroube. Comme autre épaississant, on peut également citer les dérivés cellulosiques tels que l'hydroxyéthylcellulose, la carboxyméthylcellu- lose, les dérivés de l'amidon, les dérivés d'éther de cellulose ayant des groupes ammonium quaternaires, des polysaccharides cationiques, des sels de polymères acryliques ou méthacryliques, des polyènes ou des polysiloxanes.

La phase huileuse de l'émulsion peut être constituée d'au moins une huile et/ou d'au moins une cire et représente de 5 à 50 % en poids du poids total de l'émulsion.

Parmi les huiles végétales, on peut citer l'huile de parleam, l'huile de jojoba, l'huile d'olive, l'huile d'amande douce, l'huile d'avocat, l'huile de coco, l'huile de germe de blé, l'huile de maïs, l'huile de palme, l'huile de sésame, l'huile de soja, l'huile d'argan, l'huile d'onagre, l'huile de bourrache, l'huile de pépins de raisin, l'huile de colza, l'huile de tournesol, l'huile de coton, l'huile de ricin, l'huile d'arachide et les huiles essentielles.

Parmi les huiles animales, on peut notamment citer l'huile de poisson, l'huile de vison et l'huile de tortue.

Parmi les huiles minérales, on peut citer les huiles hydrocarbonées telles que les huiles de paraffine, le squalane, la vaseline et l'isohexadécane.

Parmi les huiles synthétiques, on peut mentionner les esters tels que les palmitates d'éthyle et d'isopropyle, le palmitate d'éthyl-2-hexyle, le palmitate de 2-octyldécyle, les myristates d'alkyle tels que le myristate d'isopropyle, de butyle, de cétyle, de 2-octyldodécyl, le stéarate d'hexyle, le stéarate de butyle, le laurate d' hexyle, le laurate de 2-hexyl- décyle, l'ixononanate d'isononyle ; des huiles de silicone comme les polydiméthylsiloxanes, les polyméthylphénylsiloxa- nes ; des acides gras supérieurs tels que les triglycérides des acides octanoïque et décanoïque, le ricinoléate de cétyle, l'octanoate de stéaryle, l'acide myristique, l'acide palmitique, l'acide stéarique, l'acide béhénique, l'acide oléique, l'acide linoléique, l'acide linolénique, l'acide isostéarique ; des alcools gras tels que le cétanol, l'alcool stéarylique, l'alcool oléique : des huiles perfluorées telles que les perfluoropolyéthers.

Parmi les cires végétales, on peut notamment citer la cire de carnauba et la cire de Candellila.

Parmi les cires animales, on peut citer la cire d'abeilles, la cire de baleine et des lanolines.

Parmi les cires minérales, on peut notamment mentionner les cires microcristallines.

Parmi les cires de synthèse, on peut mentionner les cires de silicones.

Les agents liants peuvent également contenir des huiles volatiles qui s'évaporent au contact de la peau et dont la présence dans la composition cosmétique est utile car elle facilite l'étalement de la composition lors de l'application sur la peau. Parmi ces huiles, on peut notamment citer les huiles de silicone telles que l'hexaméthyldisiloxane, le cyclopen- tadiméthylsiloxane et le cyclotétradiméthylsiloxane, ainsi que les huiles fluorées telles que celle vendue par la Société Montefluos sous la dénomination de "Galden".

Les huiles et/ou cires mentionnées ci-dessus peuvent également constituer des agents liants de compositions cosmétiques anhydres et être également présentes en une proportion comprise entre 1 et 90 % et de préférence entre 5 et 80 %.

On va maintenant donner à titre d'illustration et sans aucun caractère limitatif, plusieurs exemples de compositions cosmétiques de maquillage ou pour les soins de la peau selon l'invention.

### EXEMPLE 1 : MASCARA

On prépare selon l'invention un mascara ayant la composition suivante :

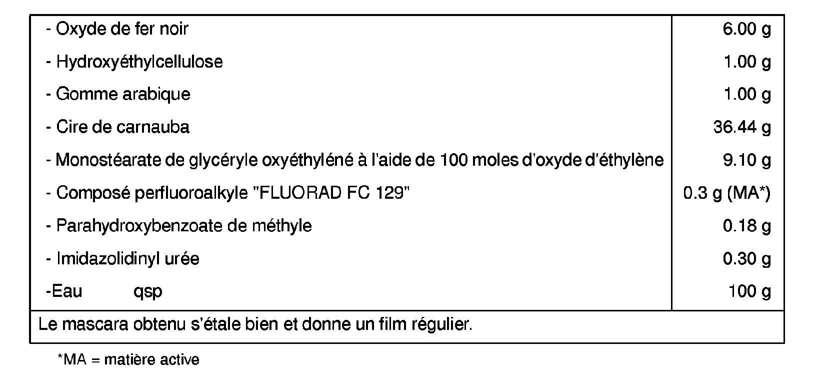

### EXEMPLE 2 : MASCARA

On prépare selon l'invention un mascara en procédant au mélange des ingrédients suivants :

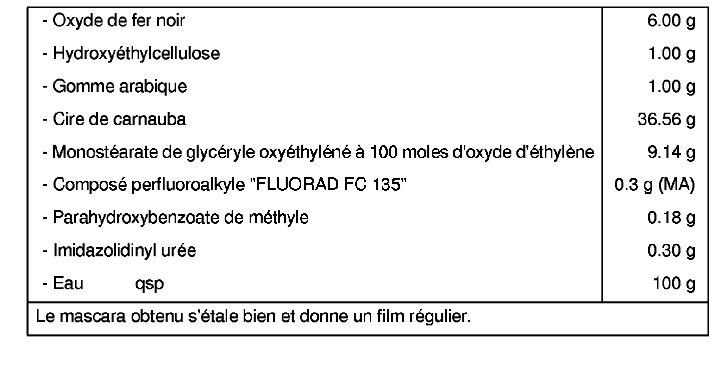

### EXEMPLE 3 : MASCARA

On prépare selon l'invention un mascara en procédant au mélange des ingrédients suivants :

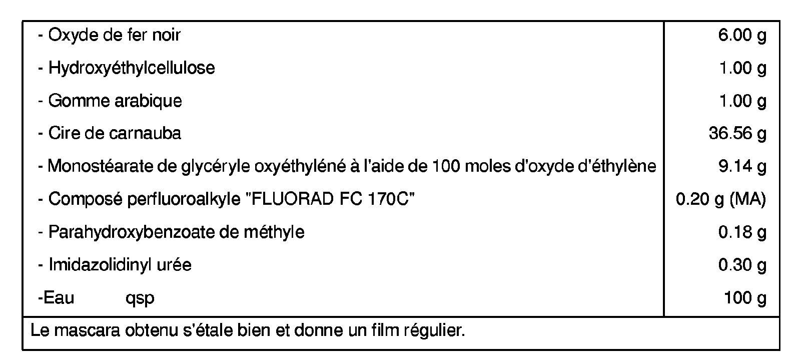

### EXEMPLE 4:MASCARA

On prépare selon l'invention un mascara en procédant au mélange des ingrédients suivants :

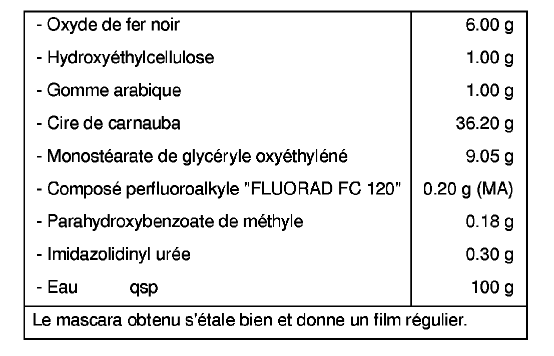

### EXEMPLE 5 : FARD A JOUES

On prépare selon l'invention un fard à joues en procédant au mélange des ingrédients suivants :

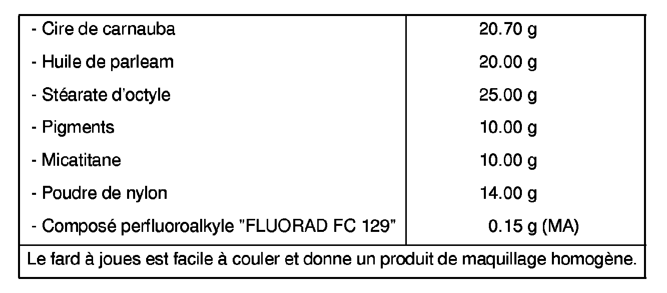

### EXEMPLE 6 : FARD A JOUES

On prépare selon l'invention un fard à joues en procédant au mélange des ingrédients suivants :

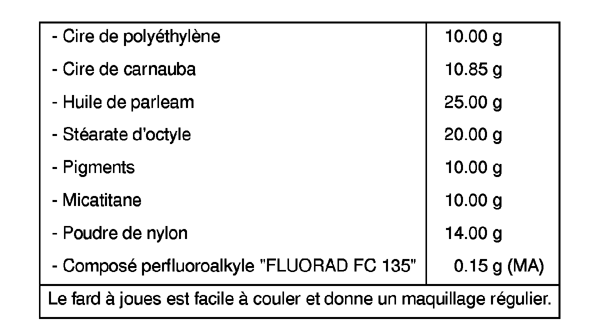

### EXEMPLE 7 : FARD A JOUES

On prépare selon l'invention un fard à joues en procédant au mélange des ingrédients suivants :

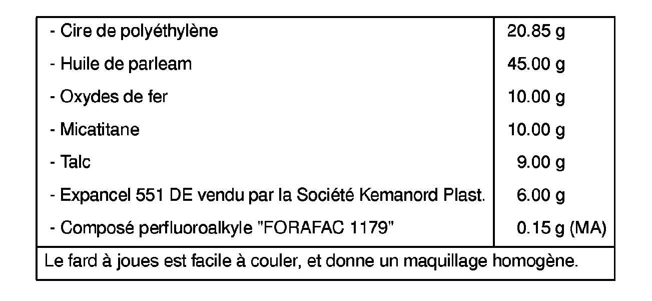

### EXEMPLE 8 : CREME TEINTEE

On prépare selon l'invention une crème teintée en procédant au mélange des ingrédients suivants :

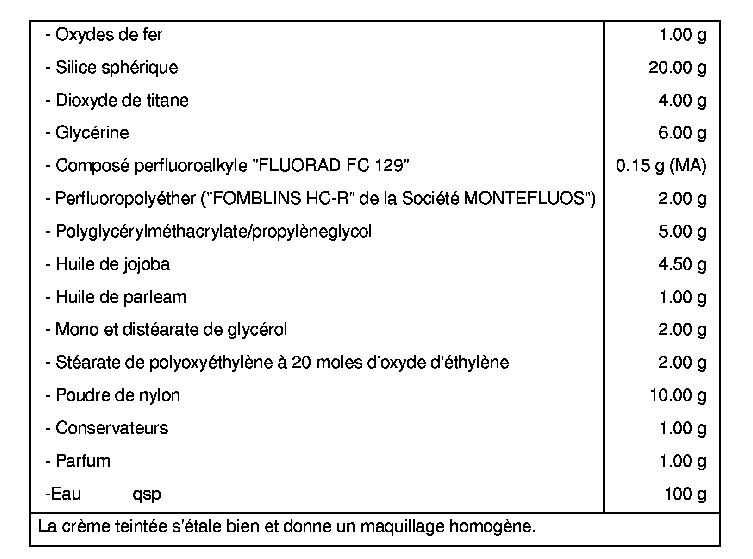

### EXEMPLE 9 : CREME TEINTEE

On prépare, selon l'invention, une crème teintée en procédant au mélange des ingrédients suivants :

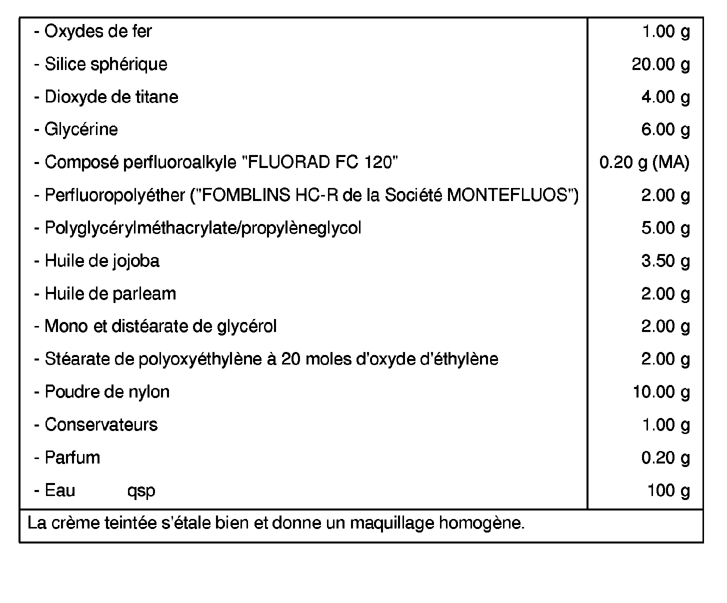

### EXEMPLE 10 : CREME TEINTEE

On prépare, selon l'invention, une crème teintée en procédant au mélange des ingrédients suivants :

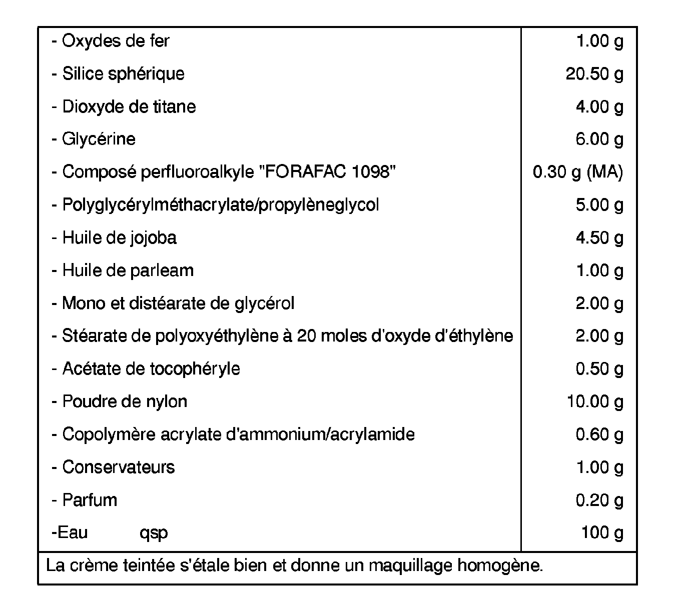

### EXEMPLE 11 : MASQUE POUR LE VISAGE

On prépare selon l'invention une émulsion huile-dans-l'eau pour le nettoyage du visage en procédant au mélange des ingrédients suivants :

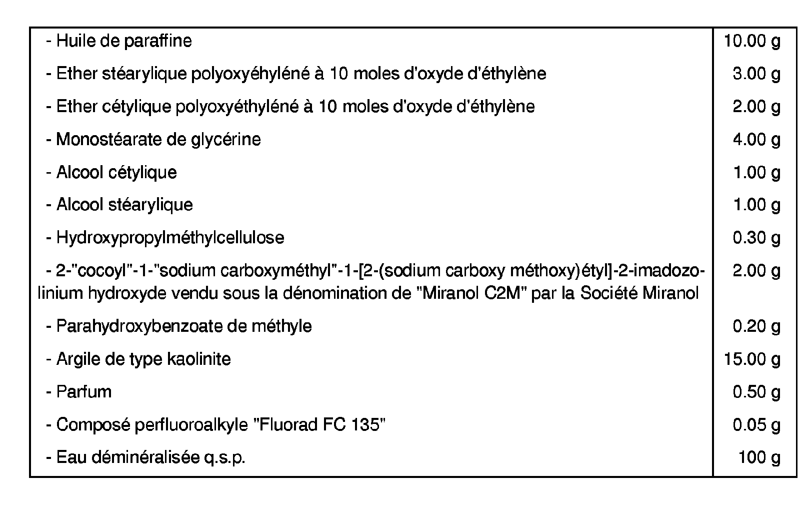

Par application de cette composition sur l'ensemble du visage à l'aide d'un léger massage et après rinçage à l'eau ou à l'aide d'un linge humide, on obtient un nettoyage parfait et l'on constate que la peau est douce et souple.

## Revendications

1. Composition cosmétique pour le maquillage ou les soins de la peau contenant de 1 à 98 % en poids d'au moins une poudre et au moins 1 % en poids d'un corps gras, caractérisée par le fait qu'elle contient en outre de 0,05 à 1 % en poids d'un composé perfluoroalkyle dont le radical alkyle a de 4 à 32 atomes de carbone, ledit composé perfluoroalkyle étant à l'état libre.

2. Composition selon la revendication 1, caractérisée par le fait que la poudre est une charge et/ou un pigment.

3. Composition selon la revendication 1 ou 2, caractérisée par le fait que la poudre a une taille de particules inférieure à 500 µm et de préférence inférieure à 100 pm.

4. Composition selon la revendication 1, caractérisée par le fait que le composé perfluoroalkyle répond à la formule suivante : 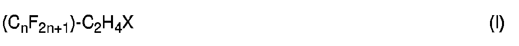 dans laquelle :
le radical CₙF₂ₙ₊₁ est linéaire ou ramifié,
n est compris entre 4 et 16,
X représente un radical choisi parmi : 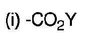 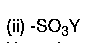
Y représentant un atome d'hydrogène, un métal alcalin
ou un groupe aminé,
(iii) -(OC₂H₄)ₘ-OH
m étant compris entre 2 et 100. 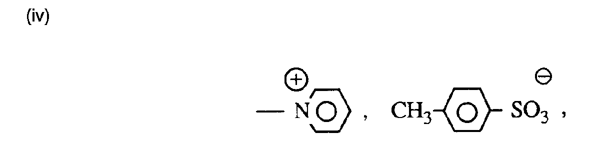 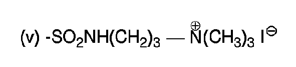 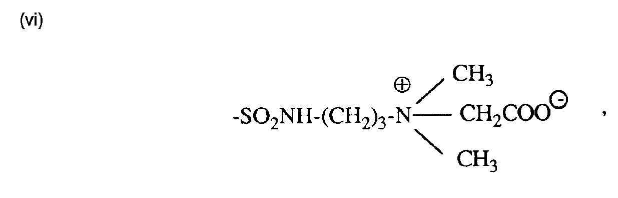 et

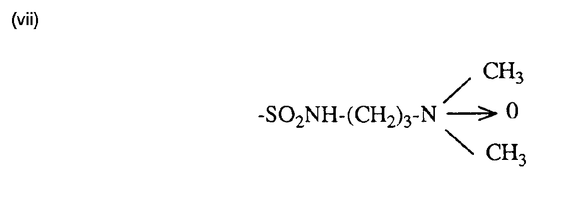

5. Composition selon la revendication 1, caractérisée par le fait que le composé perfluoroalkyle répond à la formule suivante : 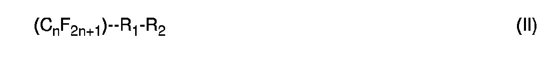 dans laquelle
le radical CₙF₂ₙ₊₁ est linéaire ou ramifié,
n est compris entre 4 et 16,
R₁ est un reste S0₃, S0₂ et CO₂,
R₂ est un reste choisi parmi:
(i) NH₄^{⊕},
(ii) - N(R₃)CH₂-CO₂⊖X⊕
R₃ étant un radical alkyle en C₁-C₄ , et
X est un atome d'hydrogène ou un métal alcalin ,
(iii) -NH(CH₂)ₚ 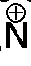 R₃)₃ l^{⊖}
p étant 1, 2, 3 ou 4 et
R₃ étant un radical alkyle en C₁-C₄ ,
(iv) -N(R₃)(CH₂CH₂0)-Y
Y étant un atome d'hydrogène ou un radical alkyle en Ci-C₄, et
R₃ étant un radical alkyle en C₁-C₄,
(v) - 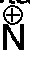 (R₃)₃
R₃ étant un radical alkyle en C₁-C₄

6. Composition cosmétique selon la revendication 4, caractérisée par le fait que le composé perfluoroalkyle de formule (I) est choisi parmi ceux de formule :

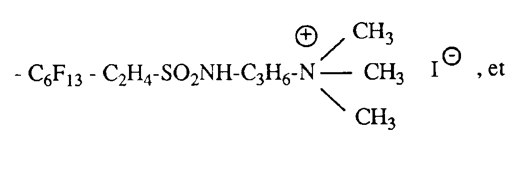

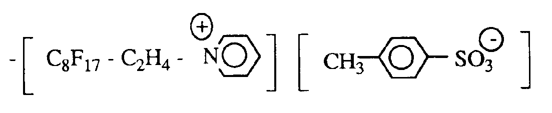

7. Composition cosmétique selon la revendication 5, caractérisée par le fait que le composé perfluoroalkyle de formule (II) est choisi parmi ceux de formule : 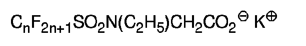
n étant ≈ 8 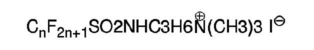
n étant 8 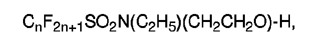 et
n étant ≈ 8 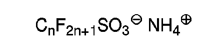
nx10

8. Composition cosmétique selon l'une quelconque des revendications précédentes, caractérisée par le fait que le composé perfluoroalkyle est de préférence présent en une proportion comprise entre 0,1 et 0,5 % en poids par rapport au poids total de la composition.

9. Composition cosmétique selon l'une quelconque des revendications précédentes, caractérisée par le fait qu'elle contient le corps gras en une proportion comprise entre 1 et 90% et de préférence entre 5 et 80 % par rapport au poids total de la composition.

10. Composition cosmétique selon l'une quelconque des revendications précédentes, caractérisée par le fait que le corps gras est une huile végétale, minérale ou animale ou une huile synthétique choisie parmi les huiles de silicones et les huiles perfluorées.

11. Composition cosmétique selon l'une quelconque des revendications 1 à 9, caractérisée par le fait que le corps gras est une cire végétale, animale ou minérale ou une cire de synthèse choisie parmi les cires de silicones.

12. Composition cosmétique selon l'une quelconque des revendications précédentes, caractérisée par le fait qu'elle se présente sous la forme d'un fard à paupières, d'un fard à joues, d'un eye-liners, d'un mascara, d'un fond de teint, d'un "blush", d'une crème teintée, d'un rouge à lèvres, d'un stick pour les lèvres ou encore d'un stick anti-cernes.

13. Composition cosmétique selon l'une quelconque des revendications précédentes, caractérisée par le fait qu'elle se présente sous forme d'une émulsion eau-dans-huile ou huile-dans-eau, la phase huile représentant de 5 à 50 % en poids du poids total de la composition.

## Claims

1. Skin-care or make-up cosmetic composition containing from 1 to 98 % by weight of at least one powder and at least 1 % by weight of a fatty substance, characterized in that it also contains from 0.05 to 1 % by weight of a perfluoroalkyl compound, the alkyl radical of which has from 4 to 32 carbon atoms, the said perfluoroalkyl compound being in the free form.

2. Composition according to Claim 1, characterized in that the powder is a filler and/or a pigment.

3. Composition according to Claim 1 or 2, characterized in that the powder has a particle size of less than 500 µm and preferably of less than 100 µm.

4. Composition according to Claim 1, characterized in that the perfluoroalkyl compound corresponds to the following formula: 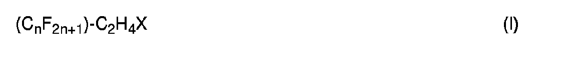 in which:
the radical CₙF₂ₙ₊₁ is linear or branched,
n is between 4 and 16,
X represents a radical chosen from: 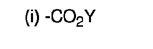 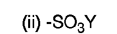
Y representing a hydrogen atom, an alkali metal
or an amino group,
(iii) -(OC₂H₄)ₘ-OH
m being between 2 and 100.

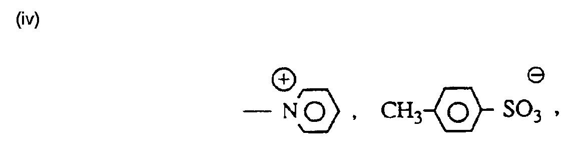

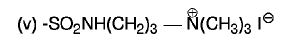

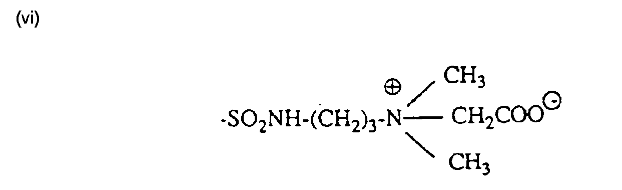

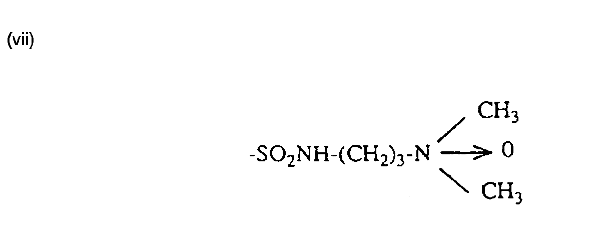

5. Composition according to Claim 1, characterized in that the perfluoroalkyl compound corresponds to the following formula: 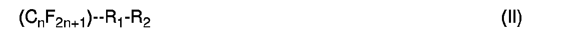 in which
the radical CₙF₂ₙ₊₁ is linear or branched,
n is between 4 and 16,
R₁ is an S0₃, S0₂ or CO₂ residue,
R₂ is a residue chosen from:
(i) NH₄^{⊕},
(ii) -N(R₃)CHₚ-CO₂⊖ X⊕
R₃ being a C₁-C₄ alkyl radical, and
X is a hydrogen atom or an alkali metal,
(iii) -NH(CH₂)pN⊕(R₃)₃ I^{⊖}
p being 1, 2, 3 or 4, and
R₃ being a C₁-C₄ alkyl radical,
(iv) -N(R₃)(CH₂CH₂O)-y
Y being a hydrogen atom or a C1-C4 alkyl radical, and
R₃ being a C₁-C₄ alkyl radical,
(V)-(R₃)₃
R₃ being a Cₗ-C₄ alkyl radical.

6. Cosmetic composition according to Claim 4, characterized in that the perfluoroalkyl compound of formula (I) is chosen from those of formula: 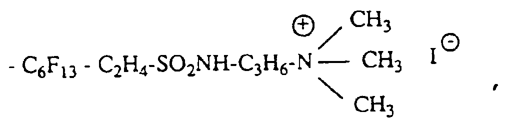 and

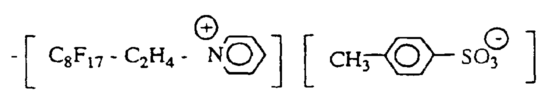

7. Cosmetic composition according to Claim 5, characterized in that the perfluoroalkyl compound of formula (II) is chosen from those of formula: 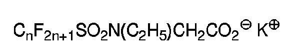 n being ≈ 8 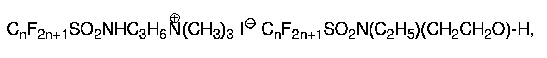 and n being 8 8

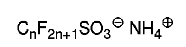

8. Cosmetic composition according to any one of the preceding claims, characterized in that the perfluoroalkyl compound is preferably present in a proportion of between 0.1 and 0.5 % by weight relative to the total weight of the composition.

9. Cosmetic composition according to any one of the preceding claims, characterized in that it contains the fatty substance in a proportion of between 1 and 90 %, and preferably of between 5 and 80 %, relative to the total weight of the composition.

10. Cosmetic composition according to any one of the preceding claims, characterized in that the fatty substance is a plant, mineral or animal oil or a synthetic oil chosen from silicone oils and perfluoro oils.

11. Cosmetic composition according to any one of Claims 1 to 9, characterized in that the fatty substance is a plant, animal or mineral wax or a synthetic wax chosen from silicone waxes.

12. Cosmetic composition according to any one of the preceding claims, characterized in that it is in the form of an eye- shadow, a facepowder, an eyeliner, a mascara, a foundation, a "blush", a tinted cream, a lipstick, a lipstick pencil or a concealer stick.

13. Cosmetic composition according to any one of the preceding claims, characterized in that it is in the form of a water- in-oil or oil-in-water emulsion, the oil phase representing from 5 to 50 % by weight of the total weight of the composition.

## Patentansprüche

1. Kosmetische Zubereitung zum Schminken oder zur Hautpflege mit einem Gehalt an 1 Gew-% bis 98 Gew.-% mindestens eines Puders und mindestens 1 Gew.-% eines Fettkörpers, dadurch gekennzeichnet, daß sie zusätzlich noch 0,5 Gew.-% bis 1 Gew.-% einer Perfluoralkylverbindung enthält, bei welcher der Alkylrest 4 bis 32 Kohlenstoffatome aufweist und die Perfluoralkylverbindung in freiem Zustand vorliegt.

2. Zubereitung nach Anspruch 1, dadurch gekennzeichnet, daß der Puder einen Trägerstoff und/oder ein Pigment darstellt.

3. Zubereitung nach Anspruch 1 oder 2, dadurch gekennzeichnnet, daß der Puder einen durchschnittlichen Teilchendurchmesser von weniger als 500 Mikrometer (µm), vorzugsweise von weniger als 100 µm aufweist.

4. Zubereitung nach Anspruch 1, dadurch gekennzeichnet, daß die Perfluoralkylverbindung der folgenden Formel entspricht: 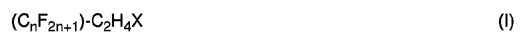 worin
der Rest CₙF₂ₙ₊₁ linear oder verzweigt ist,
n zwischen 4 und 16 sein kann,
X eine Gruppe bedeutet, welche unter den folgenden Resten ausgewählt ist: 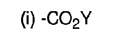 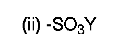
wobei Y ein Wasserstoffatom, ein Erdalkalimetall oder eine Aminogruppe bedeutet, 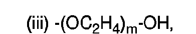
wobei m zwischen 2 und 100 bedeutet; 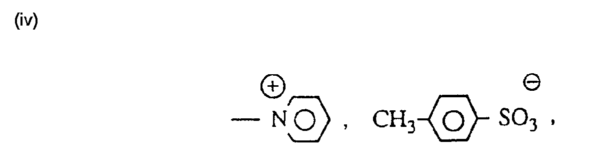 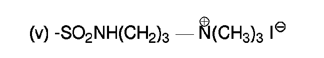 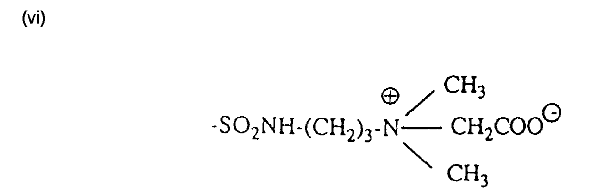 und

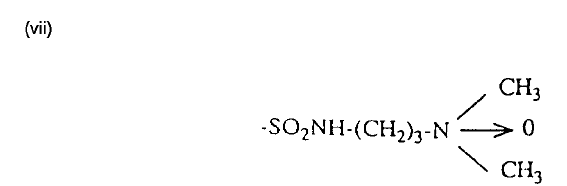

5. Zubereitung nach Anspruch 1, dadurch gekennzeichnet, daß die Perfluoralkylverbindung der folgenden Formel entspricht: 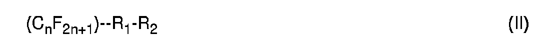 worin:
der Rest CₙF₂ₙ₊₁ linear oder verzweigt ist,
n zwischen 4 und 16 liegt,
R₁ eine Gruppe S0₃, S0₂ undd C0₂ bedeutet,
R₂ einen Rest bedeutet, welcher unter den folgenden Gruppen ausgewählt ist:
(i) NH₄⊕ ,
(ii) -N(R₃)CH₂-CO₂⊖ X⊕
wobei R₃ einen C₁-C₄-Alkylrest, und
X ein Wasserstoffatom oder ein Alkalimetall bedeuten,
(iii) -NH(CH₂)p 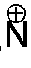 (R₃)₃ l^{⊝}
wobei p 1, 2, 3 oder 4, und
R₃ einen C₁-C₄-Alkylrest bedeuten,
(iv) -N(R₃)(CH₂CH₂0)-Y
wobei Y ein Waserstoffatom oder einen C₁-C₄-Alkylrest, und
R₃ einen C₁-C₄-Alkylrest bedeuten,
(v) - 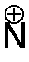 (R₃)₃
wobei R₃ einen C₁ -C₄-Alkylrest bedeutet.

6. Kosmetische Zubereitung nach Anspruch 4, dadurch gekennzeichnet, daß die Perfluoralkylverbindung gemäß der Formel (I) unter den folgenden Stoffen gemäß der Formel ausgewählt sind: 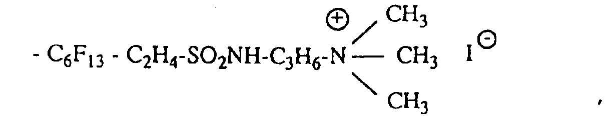 und

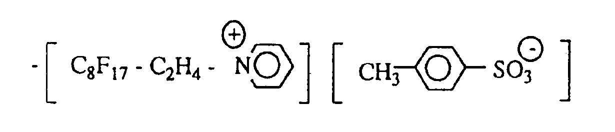

7. Kosmetische Zubereitung nach Anspruch 5, dadurch gekennzeichnet, daß die Perfluoralkylverbindung gemäß der Formel (I I) unter den folgenden Stoffen gemäß der Formel ausgewählt sind: 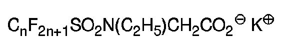
wobei n 8 bedeutet, 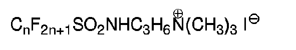
wobei n ≈ 8 bedeutet, 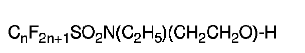
, und 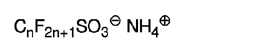
wobei n ≈ 10 bedeutet.

8. Kosmetische Zubereitung nach einem der vorstehenden Ansprüche, dadurch gekennzeichnet, daß die Perfluoralkylverbindung vorzugsweise in einem Mengenverhältnis im Bereich zwischen 0,1 Gew.-% und 0,5 Gew.-% in bezug auf das Gesamtgewicht der Zubereitung enthalten ist.

9. Kosmetische Zubereitung nach einem der vorstehenden Ansprüche, dadurch gekennzeichnet, daß sie den Fettkörper in einem Mengenverhältnis im Bereich zwischen 1 Gew.-% und 90 Gew.-%, vorzugsweise zwischen 5 Gew.-% und 80 Gew.-% in bezug auf das Gesamtgewicht der Zubereitung enthalten ist.

10. Kosmetische Zubereitung nach einem der vorstehenden Ansprüche, dadurch gekennzeichnet, daß der Fettkörper ein pflanzliches, mineralisches oder tierisches oder auch synthetisches Öl darstellt, welches unter den Silikonölen und perfluorierten Ölen ausgewählt ist.

11. Kosmetische Zubereitung nach einem der Ansprüche 1 bis 9, dadurch gekennzeichnet, daß der Fettkörper ein pflanzliches, tierisches oder mineralisches Wachs oder auch ein synthetisches Wachs darstellt, welches unter den Silikonwachsen ausgewählt ist.

12. Kosmetische Zubereitung nach einem der vorstehenden Ansprüche, dadurch gekennzeichnet, daß sie in Form einer Lidschminke, eines Make-up, eines Eyeliners, einer Wimperntusche, einer Teintgrundlage, von Wangenrouge ("blush"), einer Teintcreme, Lippenrouge, Lippenstift oder auch eines Stifts gegen Augenringe vorliegt.

13. Kosmetische Zubereitung nach einem der vorstehenden Ansprüche, dadurch gekennzeichnet, daß sie in Form einer Wasser-in-Öl-Emulsion oder einer Öl.in.Wasser.Emulsion vorliegt, wobei die Ölphase 5 Gew.-% bis 50 Gew.- % in bezug auf das Gesamtgewicht der Zusammensetzung darstellt.
